# FASCICULE DE BREVET EUROPEEN

(11) **EP 2 826 511 B1**
(45) Date de publication et mention de la délivrance du brevet: **06.01.2016**
(21) Numéro de dépôt: 14175638.7
(22) Date de dépôt: 03.07.2014
(51) Int. Cl.: A61M 16/00

(54) **Appareil d'assistance respiratoire avec estimation du débit de gaz sortant par la valve expiratoire**
Gerät zur Atmungsunterstützung mit Abschätzung der Durchflussmenge an Gas, das aus dem Ausatemventil austritt
Breathing assistance device with estimation of the flow rate of gas exiting by the exhalation valve

(30) Priorité: 19.07.2013 FR 1357129
(43) Date de publication de la demande: 21.01.2015
(73) Titulaire: Air Liquide Medical Systems, 92160 Antony (FR)
(72) Inventeur: Morin, Graziella, 75014 Paris (FR)
(74) Mandataire: Pittis, Olivier

(56) Documents cités:
- WO-A2-2011/060204
- FR-A1- 2 829 942
- US-A- 5 129 390
- US-A- 5 390 666
- US-A1- 2010 078 026

## Description

L'invention concerne un appareil de ventilation artificielle ou ventilateur médical d'un patient permettant une estimation des fuites d'air non intentionnelles entre l'appareil de ventilation artificielle et un patient alimenté en air par ledit appareil de ventilation.

Les dispositifs ou appareils de ventilation artificielle comportant une valve expiratoire présentent usuellement un moyen de mesure du débit sortant de la valve expiratoire ou un moyen de mesure du débit patient, c'est-à-dire du débit proximal.

En effet, en l'absence d'un tel moyen de mesure du débit sortant de la valve expiratoire, ces dispositifs sont dans l'impossibilité de monitorer de nombreuses données utiles au médecin pour surveiller le bon déroulement du traitement, par exemple l'apparition de fuites entre l'appareil et le patient, et connaitre par ailleurs la ventilation du patient, par exemple son débit respiratoire.

Ainsi, si l'estimation des fuites est un monitorage assez bien maitrisé lorsque le débit entrant et le débit sortant du système, c'est-à-dire appareil et accessoires (circuit, humidificateur...), sont connus, il n'en va pas de même avec les appareils de ventilation artificielle à circuit à valve expiratoire qui sont conçus pour pouvoir fonctionner sans mesure de débit expiratoire.

Le document US 2010/078026 divulgue un appareil de ventilation artificielle d'un patient par comprenant un capteur de débit, lequel est configuré pour mesurer le débit du gaz expiré par le patient.

En effet, la difficulté technique réside en l'absence de mesure du débit gazeux au niveau de la valve expiratoire puisqu'il faut réussir à différencier une fuite non intentionnelle potentielle du débit gazeux sortant par ladite valve expiratoire.

L'estimation des fuites devient alors beaucoup plus délicate puisqu'il est impossible de savoir quelle proportion du débit délivré par la machine de ventilation s'échappe via une fuite potentielle ou va au patient, et par ailleurs quelle proportion du débit expiré par le patient s'échappe par la valve expiratoire ou par une fuite.

Pour ces appareils avec circuit à valve expiratoire et à simple branche, la connaissance du débit sortant de la valve expiratoire se fait actuellement via l'ajout d'un module supplémentaire de mesure de débit et/ou de pression proximaux.

Or, les inconvénients d'un tel module supplémentaire de mesure sont notamment le poids qu'il exerce au niveau de la bouche du patient qui engendre une gène, une déformation du visage..., et l'ajout indispensable de fils supplémentaires entre l'appareil et le module/patient qui crée une nuisance et un encombrement...

En résumé, à ce jour, l'estimation du débit de fuite et la maitrise du débit patient existent donc uniquement sur les appareils de ventilation à double branche avec mesure de débit expiratoire, sur ceux à simple branche sans valve expiratoire et sur ceux à simple branche à valve expiratoire avec module supplémentaire à capteurs de pression et/ou débit proximaux.

Le problème qui se pose est dès lors de pouvoir obtenir aisément une estimation du débit sortant de la valve expiratoire d'un appareil de ventilation artificielle en l'absence de mesure au niveau de celle-ci et/ou de capteur(s) proximal, de manière à pouvoir ensuite utiliser cette estimation du débit pour estimer et suivre par exemple le débit de fuite de l'appareil ou le débit respiratoire du patient

En d'autres termes, le but de l'invention est de proposer un appareil de ventilation artificielle amélioré, avec circuit de gaz à valve expiratoire, qui soit apte à et conçu pour réaliser, lors de son utilisation, une estimation du débit de la valve expiratoire et ce, sans mesure du débit expiratoire, que l'appareil soit à simple branche ou à double branche, et sans utiliser par ailleurs de module supplémentaire de mesure de débit et/ou de pression proximaux venant se raccorder audit appareil.

La solution de l'invention concerne alors un appareil de ventilation artificielle ou ventilateur d'un patient, c'est-à-dire un être humain, comprenant :
- une source de gaz apte à et conçue pour délivrer un gaz respiratoire,
- un circuit patient comprenant au moins une branche inspiratoire reliée fluidiquement à la source de gaz pour recevoir le débit de gaz respiratoire délivré par la source de gaz,
- une valve expiratoire, agencée sur ou reliée au circuit patient, dont l'ouverture et la fermeture sont commandées par une ligne de pressurisation reliée fluidiquement à la source de gaz,
- au moins une électrovanne agencée sur ladite ligne de pressurisation et commandée par des moyens de pilotage de manière à autoriser ou à interdire une transmission de pression à la valve expiratoire, via ladite ligne de pressurisation,
- des moyens de pilotage conçus pour délivrer au moins un signal de tension U à ladite au moins une électrovanne pour commander l'électrovanne de manière à autoriser ou interdire la transmission de pression à la valve expiratoire au travers de ladite ligne de pressurisation et à ainsi commander la valve expiratoire, en fonction dudit au moins un signal de tension U, entre au moins une position totalement ouverte, une position totalement fermée et une ou plusieurs positions intermédiaires, et
- un moyen de mesure de pression agencé de manière à mesurer la pression P du gaz délivré par la source de gaz,
caractérisé en ce qu'il comprend en outre des moyens à microprocesseurs mettant en oeuvre au moins un algorithme permettant de déterminer le débit de gaz Qve sortant par la valve expiratoire à partir d'au moins un signal de pression P délivré par le moyen de mesure de pression et dudit au moins un signal de tension U délivré par les moyens de pilotage.

On comprend que, selon l'invention, le débit de gaz sortant par la valve expiratoire est obtenu non pas par mesure directe dudit débit au niveau de ladite valve expiratoire mais est estimé à partir d'un (ou plusieurs) signal de pression P mesuré par un capteur de pression et d'un (ou plusieurs) signal de tension U délivré par les moyens de pilotage du ventilateur, à savoir typiquement une carte électronique, par exemple du type à microcontrôleur et algorithme.

En effet, selon l'invention, on caractérise l'ensemble circuit patient et valve expiratoire en fonction de la commande de la valve expiratoire (tension) et des mesures de pression et de débit.

Comme expliqué en détail ci-après, le modèle est paramétré pour chaque valve par une manoeuvre à opérer et des enregistrements.

Il faut effectuer la manoeuvre à chaque changement de configuration de valve car la caractérisation peut varier fortement entre deux valves expiratoires, même si elles sont du même modèle.

La mise en place du modèle de caractérisation comporte deux étapes, à savoir :
a) une détermination de la tension de fermeture de la valve expiratoire, et
b) un enregistrement d'une table de points de fonctionnements de la valve, à pression, tension et débit de valve non nuls.

En cours de ventilation, l'estimation du débit sortant par la valve s'effectue en deux étapes, à savoir :
i) détermination si le débit est nul ou non,
ii) le cas échéant, double interpolation en fonction de la pression mesurée à un instant t et de la tension de commande à l'instant t pour en déduire le débit sortant de l'appareil à l'instant t.

Bien que le modèle de l'invention ne soit qu'approximatif, étant donné que les résultats dépendent de la position physique de la valve expiratoire et d'autres paramètres, notamment la température, il permet cependant d'obtenir une bonne estimation du débit de gaz Qve sortant par la valve expiratoire.

Les avantages majeurs de l'invention sont que, d'une part, l'estimation du débit de gaz Qve sortant par la valve expiratoire se fait sans avoir recours à une mesure au niveau de ladite valve expiratoire, donc sans avoir à intégrer un capteur à ce niveau, même lorsque l'appareil est à branche unique, et, d'autre part, sans ajout de modèle de mesure supplémentaire venant se raccorder à l'appareil.

Selon le cas, l'appareil de ventilation artificielle de l'invention peut comprendre l'une ou plusieurs des caractéristiques techniques suivantes :
- il comprend en outre un moyen de mesure de débit agencé de manière à mesurer le débit Q du gaz délivré par la source de gaz.
- la valve expiratoire comprend un ballonnet à volume variable, c'est-à-dire qui peut être gonflé ou dégonflé, ou une membrane déformable de manière à contrôler la sortie de gaz via ladite valve expiratoire par le biais de ce ballonnet ou de cette membrane déformable.
- la ligne de pressurisation amène une pression et/ou un débit de gaz qui va agir sur le ballonnet ou sur la membrane déformable de la valve expiratoire qui commande et/ou contrôle l'ouverture ou la fermeture de ladite valve expiratoire.
- les moyens de pilotage, le moyen de mesure de débit et le moyen de mesure de pression coopèrent avec les moyens à microprocesseurs de manière à paramétrer l'algorithme des moyens à microprocesseurs servant à déterminer le débit Qve de gaz sortant par la valve expiratoire pour une pression P et une tension U données.
- il comprend en outre des moyens de mémorisation pour mémoriser au moins les valeurs de tension U, de débit Q et de pression P issues des moyens de pilotage, du moyen de mesure de débit et du moyen de mesure de pression, respectivement.
- les moyens à microprocesseurs coopèrent avec les moyens de mémorisation de manière à retrouver des valeurs de tension U, de débit Q et de pression P utilisées par l'algorithme pour estimer un débit de gaz Qve donné sortant par la valve expiratoire.
- les moyens de pilotage sont aptes à et conçus pour délivrer des valeurs de tension U décroissantes ou croissantes à partir de la valeur de tension d'ouverture maximale de la valve de manière à fermer progressivement la valve expiratoire.
- lorsque la branche inspiratoire est obturée au niveau de la connexion du patient :
   a) la source de gaz est apte à délivrer du gaz à pression donnée P,
   b) les moyens de pilotage sont programmés pour délivrer des valeurs de tension U₁, U₂... Uₙ décroissantes ou croissantes à partir de la valeur de tension d'ouverture maximale de la valve de manière à fermer progressivement la valve expiratoire,
   c) le capteur de débit est apte à et conçu pour mesurer des valeurs de débit de gaz données Q'₁, Q'₂... Q'ₙ correspondant au débit de gaz sortant par la valve expiratoire, et
   d) les moyens à microprocesseurs permettent de déterminer la tension de commande de fermeture Uferm au-dessus ou en-dessous de laquelle le débit de gaz

Qve sortant par la valve expiratoire est nul.
- les moyens de mémorisation permettent de mémoriser la valeur de tension de fermeture Uferm correspondant à l'instant donné t où le débit Q délivré par la source de gaz est nul, c'est-à-dire lorsque l'on a : Q = 0.
- les moyens de pilotage sont aptes à déterminer si le débit Qve de gaz sortant de la valve expiratoire est nul à partir de la valeur de tension instantanée Ui à l'aide de la valeur de tension de fermeture Uferm mémorisée.
- les moyens de pilotage sont conçus pour délivrer au moins un signal de tension à l'électrovanne pour commander l'électrovanne de manière à autoriser ou interdire la transmission de pression à la valve expiratoire au travers de ladite ligne de pressurisation et à ainsi commander la valve expiratoire entre au moins :
   - une position totalement ouverte correspondant à une valeur de tension de commande maximale non nulle (Umax) de l'électrovanne et dans laquelle le débit de gaz Qve sortant par la valve expiratoire est maximal,
   - une position totalement fermée correspondant à une valeur de tension de commande de fermeture Uferm de l'électrovanne, avec Umax > Uferm ≥ 0, et dans laquelle le débit de gaz Qve sortant par la valve expiratoire est nul,
   - une ou plusieurs positions intermédiaire correspondant à une ou plusieurs valeurs de tension de commande intermédiaires Uint comprises entre Umax et Uferm, et dans laquelle ou lesquelles le débit de gaz Qve sortant par la valve expiratoire est inférieur au débit maximal et non nul.
      - lorsque la branche inspiratoire est obturée au niveau de la connexion du patient :
         i) la source de gaz est apte à délivrer plusieurs valeurs de pression de gaz successives P₁, P_{2...} Pⱼ ...Pₚ,
         ii) les moyens de pilotage sont programmés pour délivrer des valeurs de tension U₁, U₂... Uᵢ ... Uₙ décroissantes ou croissantes comprises depuis la valeur de tension d'ouverture maximale de la valve jusqu'à la valeur de tension de fermeture Uferm, pour chaque valeur de pression de gaz successive Pj délivrée par la source de gaz,
         iii) le capteur de débit est apte à et conçu pour mesurer des valeurs de débit de gaz données (Q_{1,1}, Q_{1,2}... Q_{i,j}... Q_{n,p}) correspondant au débit sortant de la valve expiratoire pour chaque valeur de pression de gaz successive P₁, P_{2...}Pₚ du gaz délivré par la source de gaz et pour chaque valeur de tension décroissantes U_{1,} U₂... Uₙ délivrée par les moyens de pilotage, et
         iv) les moyens de mémorisation sont aptes à et conçu pour mémoriser les valeurs de débit de gaz (Q_{1,1}, Q_{1,2}... Q_{i,1}... Q_{n,p}) mesurées par le capteur de débit de manière associée aux valeurs de pression de gaz successives P₁, P₂...Pₚ et auxdites valeurs de tensions croissantes ou décroissantes U₁, U₂... Uₙ correspondantes.
      - les moyens de pilotage sont aptes à calculer le débit Qve de gaz sortant de la valve expiratoire à partir des valeurs de débit de gaz (Q_{1,1}, Q_{1,2}... Q_{i,j}... Q_{n,p}) mesurées par le capteur de débit et mémorisées par les moyens de mémorisation pour une valeur de tension instantanée Ui et pour une valeur de pression instantanée mesurée de gaz Pj, à l'aide d'une double interpolation sur une table (Ui, Pj, Qi,j) mémorisée au sein desdits moyens de mémorisation.
      - le moyen de mesure de débit est un capteur de débit.
      - le moyen de mesure de pression est un capteur de pression.
      - les moyens de pilotage comprennent une carte électronique, de préférence du type à microcontrôleur et algorithme.
      - les moyens de mémorisation comprennent une mémoire de stockage.
      - les moyens de mémorisation comprennent au moins une mémoire de stockage type RAM, EEPROM ou Flash.
      - la branche inspiratoire est reliée fluidiquement à une interface patient, de préférence l'interface patient comprend un masque respiratoire, une sonde de trachéotomie ou une sonde d'intubation.
      - la source de gaz est une turbine, un récipient de gaz sous pression ou une ligne d'alimentation en gaz sous pression alimentant une prise murale ou analogue, de préférence une turbine.
      - la source de gaz délivre de l'air, de l'oxygène ou de l'air enrichi en oxygène.
      - le circuit patient comprenant une branche inspiratoire et une branche expiratoire, la valve expiratoire étant agencée sur la branche expiratoire.
      - la valve expiratoire est agencée du côté de l'extrémité aval de la branche expiratoire située du côté de ou dans l'appareil.
      - il comprend des moyens d'alimentation électrique, notamment une prise et un cordon de raccordement permettant de brancher l'appareil au réseau électrique du secteur de manière à lui fournir du courant électrique, ou au moins une batterie ou analogue.

L'invention va maintenant être mieux comprise grâce à la description détaillée suivante faite en références aux Figures annexées parmi lesquelles :
- la Figure 1 représente un premier mode de réalisation d'un appareil de ventilation artificielle selon l'invention avec circuit patient à une branche respiratoire;
- la Figure 2 représente un deuxième mode de réalisation d'un appareil de ventilation artificielle selon l'invention avec circuit patient à deux branches respiratoires;
- la Figure 3 détaille le premier mode de réalisation de la Figure 1; et
- la Figure 4 illustre les étapes de caractérisation de la valve expiratoire.

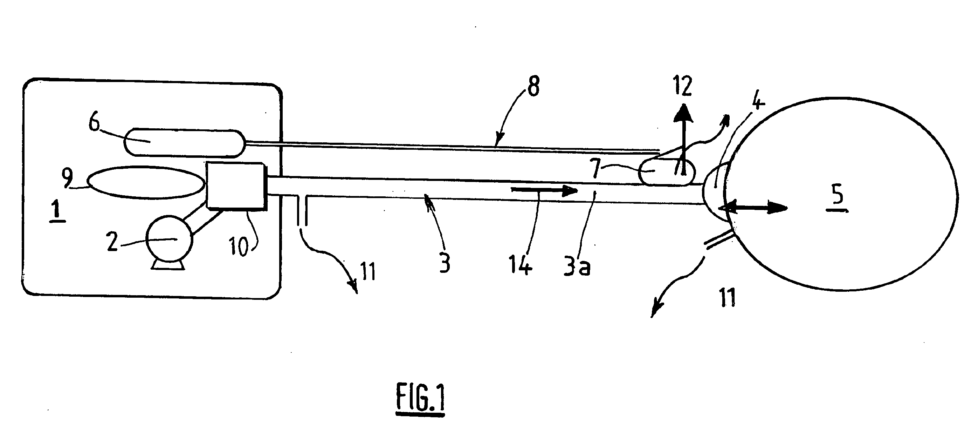

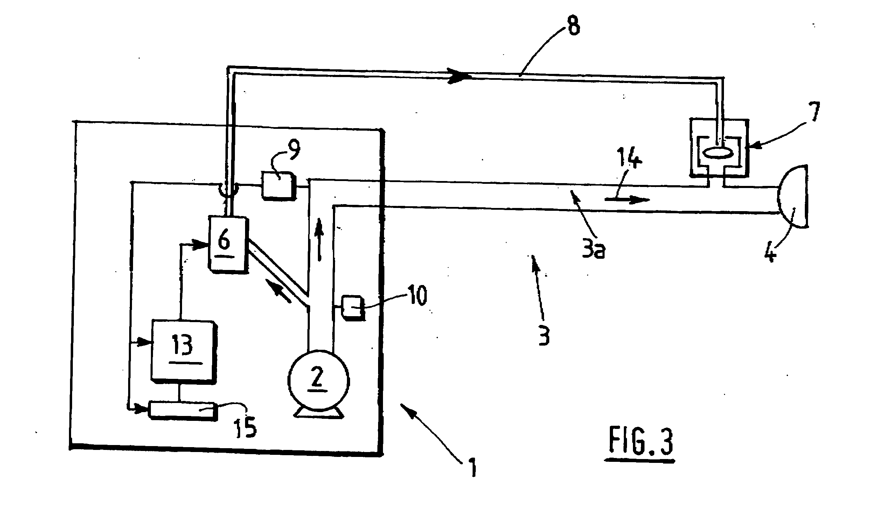

Les Figures 1 et 3 représentent un premier mode de réalisation d'un appareil 1 de ventilation artificielle, c'est-à-dire un ventilateur médical, tel par exemple le ventilateur Monnal T50 commercialisé par la Demanderesse, comprenant, selon la présente invention, un circuit patient 3 à branche respiratoire unique 3a, encore appelée branche inspiratoire, qui est alimenté fluidiquement par une source de gaz 2, telle une turbine ou micro-soufflante, ou un réseau de gaz mural relié fluidiquement au ventilateur 1, de manière à délivrer un débit de gaz sous pression, tel de l'air, c'est-à-dire à une pression supérieure la pression atmosphérique (i.e. > 1 atm), à un patient 5.

Le circuit patient 3 permet de convoyer le gaz issu de la source de gaz 2 jusqu'à une interface patient 4, tel un masque ou une sonde trachéale ou d'intubation, qui permet de délivrer le débit de gaz au patient 5. Le sens du flux de gaz est schématisé par la flèche 14.

Des moyens de commande de valve comprenant un conduit de pressurisation 8 sont conçus pour et aptes à commander fluidiquement une valve expiratoire 7 agencée sur le circuit patient 3, à proximité de l'interface patient 4, à savoir par exemple un masque nasal ou facial, ou une sonde d'intubation ou trachéale.

La valve expiratoire 7 est apte à être commandée entre plusieurs positions d'ouverture/fermeture, dont au moins une position dite « totalement ouverte » dans laquelle du gaz s'échappe à un débit maximum donné par ladite valve expiratoire 7 de manière à évacuer un maximum de gaz vers l'atmosphère ambiante (en 12), et au moins une position dite «fermée» dans laquelle aucun flux de gaz ne s'échappe par ladite valve expiratoire 7, c'est-à-dire que le gaz est retenu dans le circuit patient 3 et dans le masque 4, comme expliqué ci-après. La valve expiratoire 7 peut en outre être commandée entre une ou plusieurs autres positions d'ouverture/fermeture dites positions « intermédiaires » conduisant à une ouverture de la valve expiratoire entre la position dite « totalement ouverte » et la position dite « fermée ».

Comme illustré sur les Figures 1 et 3, les moyens de détermination de débit 9, tel un capteur de débit, sont agencés en sortie de la source de gaz 2 et permettent de déterminer le débit de gaz Q sortant de la source de gaz 2. Le capteur de débit peut être par exemple un capteur de référence commerciale AWM700 Series de la société Honeywell.

En outre, un moyen de mesure de pression 10, de préférence un capteur de pression, apte à mesurer la pression P du gaz délivré par la source de gaz 2 est agencé sur la branche inspiratoire 3a, de préférence en amont de celle-ci, c'est-à-dire du côté du ventilateur 1.

Une électrovanne 6 ou un bloc de plusieurs électrovannes est agencé(e) sur la ligne de pressurisation 8 et est commandé(e) par des moyens de pilotage 13 de manière à autoriser ou à interdire une transmission de pression à la valve expiratoire 7, via ladite ligne de pressurisation 8, c'est-à-dire de manière à commander une ouverture partielle ou totale de ladite valve expiratoire 7 pour autoriser un passage de débit de gaz ou, à l'inverse, pour ferme ladite valve expiratoire 7 et donc empêcher toute sortie de gaz.

En fait, la ligne de pressurisation 8 amène une pression et/ou un débit de gaz qui va agir sur le ballonnet de la valve expiratoire 7 qui commande l'ouverture ou la fermeture de cette valve 7.

Plus précisément, les moyens de pilotage 13 sont conçus pour délivrer au moins un signal de tension U à ladite au moins une électrovanne 6 pour la commander de manière à autoriser ou interdire la transmission de pression à la valve expiratoire 7 au travers de ladite ligne de pressurisation 8 et à ainsi commander la valve expiratoire 7, en fonction du au moins un signal de tension U délivré par lesdits moyens de pilotage, entre au moins la position totalement ouverte, la position totalement fermée et une ou plusieurs positions intermédiaires.

Préférentiellement, les moyens de pilotage 13 comprennent une carte électronique comprenant un microcontrôleur à algorithme(s).

Selon l'invention, le ventilateur 1 comprend en outre des moyens à microprocesseurs mettant en oeuvre au moins un algorithme permettant de déterminer le débit de gaz Qve sortant par la valve expiratoire 7 à partir d'au moins un signal de pression P délivré par le moyen de mesure de pression 10 et d'au moins un signal de tension U délivré par les moyens de pilotage 13.

Les moyens de pilotage 13, le moyen de mesure de débit 9 et le moyen de mesure de pression 10 coopèrent avec les moyens à microprocesseurs de manière à paramétrer l'algorithme servant à déterminer le débit Qve de gaz sortant par la valve expiratoire 7 pour une pression P et une tension U données, puis ensuite le débit de fuite de la valve expiratoire 7.

Le ventilateur 1 comprend en outre des moyens de mémorisation 15, tel une mémoire de stockage de données, permettant de mémoriser au moins les valeurs de tension U, de débit Q et de pression P issues des moyens de pilotage 13, du moyen de mesure de débit 9 et du moyen de mesure de pression 10, respectivement.

En fait, les moyens à microprocesseurs coopèrent avec les moyens de mémorisation 15 de manière à retrouver des valeurs de tension U, de débit Q et de pression P utilisées par l'algorithme pour estimer le débit de gaz Qve sortant par la valve expiratoire 7.

### Modèle de caractérisation de la valve expiratoire

La caractérisation de la valve expiratoire est effectuée pendant l'autotest de l'appareil de ventilation par exemple, qui consiste à faire une série de vérifications techniques avant de lancer la ventilation proprement dite.

L'autotest s'effectue avec le circuit patient 3 obturé à son extrémité par un bouchon ou analogue, c'est-à-dire avec branche inspiratoire 3a obturée au niveau de l'interface 4 (Fig. 1). Le patient 5 n'est donc pas raccordé à l'appareil à ce moment.

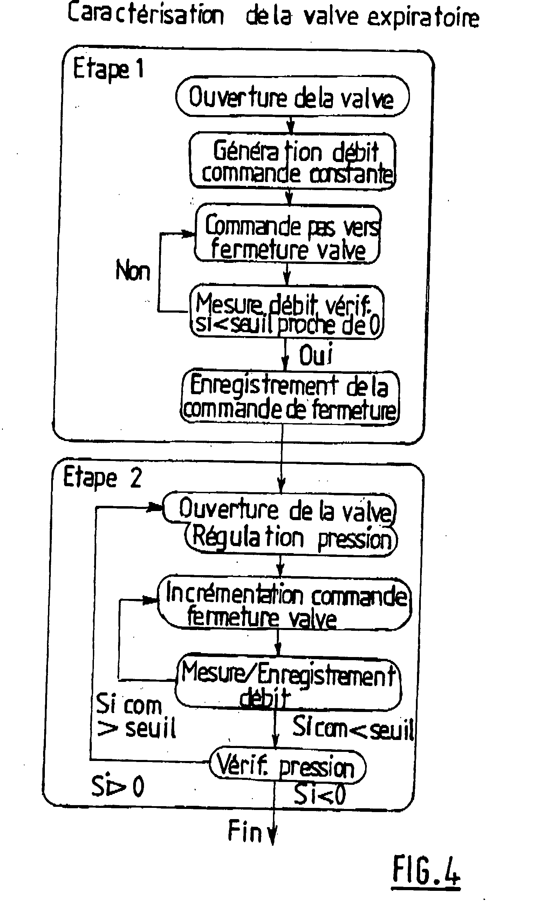

La caractérisation de la valve expiratoire 7 se déroule en deux étapes successives, comme illustré en Figure 4 :
- Etape 1 : tout d'abord, on opère une recherche du point de commande de fermeture de la valve expiratoire 7. Pour ce faire, la valve expiratoire 7 est en position ouverte. Dans l'appareil, on génère un débit de gaz avec une commande fixe de la turbine 2 et on ferme progressivement la valve expiratoire 7 jusqu'à mesurer un débit nul. Cette fermeture progressive se fait en ouvrant progressivement l'électrovanne 6 qui va pressuriser progressivement , via la ligne de pressurisation 8 le ballonnet de la valve expiratoire 7. Lorsqu'on mesure un débit nul, on enregistre la valeur de commande de la valve expiratoire 7 correspondant à ce débit nul.
- Etape 2 : ensuite, on établit un tableau incluant les valeurs de débit sortant de la valve expiratoire 7 en fonction de la commande (tension) de ladite valve expiratoire 7 et de la pression générée par la turbine 2 de l'appareil. Pour ce faire, on ouvre d'abord la valve expiratoire 7 et on régule la turbine 2 pour qu'elle délivre une pression de gaz donnée, par exemple successivement 15, 10, 5 et 2 cm H₂O, et on mesure le débit en fonction de différents paliers de commande de la valve, c'est-à-dire de la tension maximale à la valeur de fermeture déterminée juste avant. On enregistre alors le débit sortant de la valve.

Au moment de l'ouverture de la valve et des fermeture partielles successives, la pression mesurée change, il faut donc attendre que la régulation de la turbine fasse son effet et que la pression mesurée soit de nouveau correcte pour relever les mesures de pression, tension et débit.

Ceci termine l'étape 2 de l'autotest.

Par la suite, le débit sortant de la valve 7 sera estimé en faisant une double interpolation en fonction de la pression mesurée et de la commande de la valve.

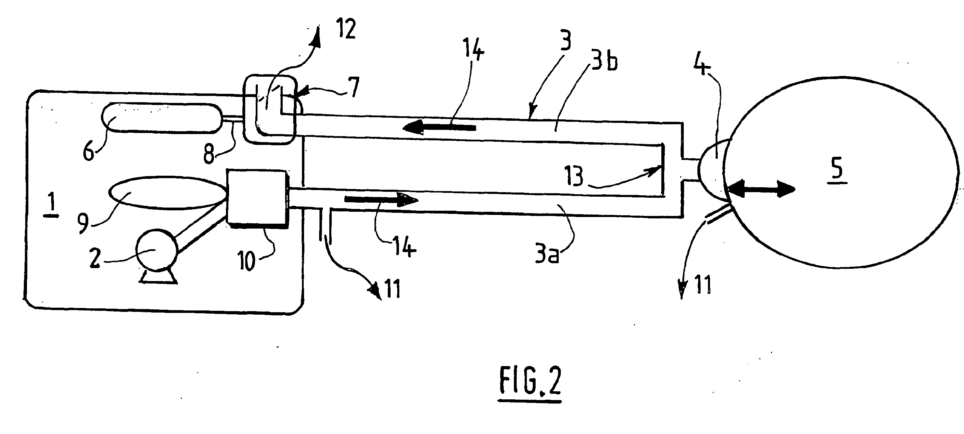

Selon l'invention, la caractérisation précédente peut être effectuée sur un circuit de gaz 3 à simple branche (branche unique : Fig. 1) et en double branche (Fig. 2). En fait, l'estimation du débit Ev est possible après l'autotest et ce, dans deux configurations de circuit possibles, à savoir à simple branche et à double branche.

Pour ce faire, les moyens de pilotage 13 délivrent des valeurs de tension U, décroissantes ou croissantes, à partir de la valeur de tension d'ouverture maximale de la valve de manière à fermer progressivement la valve expiratoire 7.

Les moyens de détermination de débit ou capteur de débit 9 permettent en fait de déterminer le débit Q de gaz sortant de la source 2 de gaz à l'instant t où les moyens de commande 6 commandent la valve expiratoire 7 en position fermée, empêchant ainsi toute sortie du gaz du circuit 3, et lorsque, dans le même temps, l'interface patient 4 ne délivre aucun débit de gaz, c'est-à-dire que l'interface 4 n'alimente pas le patient en gaz respiratoire et qui est obstruée par un bouchon.

Ceci peut être obtenu en obturant le circuit patient 3 au niveau de l'interface 4 alimentant le patient 5. Le débit de gaz déterminé par les moyens de détermination de débit 9 dans cette configuration correspond alors à une valeur de débit de fuite non intentionnelle.

En effet, lorsque la branche inspiratoire 3a est obturée au niveau de l'interface 4 du patient 5, la source de gaz 2 délivre du gaz à pression P donnée, les moyens de pilotage 13 sont programmés pour délivrer des valeurs de tension U₁, U₂... Uₙ décroissantes ou croissantes à partir de la valeur de tension d'ouverture maximale Umax de la valve 7 expiratoire de manière à fermer progressivement la valve expiratoire 7, le capteur de débit 9 mesure des valeurs de débit de gaz données (Q'₁, Q'₂... Q'ₙ) correspondant au débit de gaz sortant par la valve expiratoire 7, et les moyens à microprocesseurs déterminent la tension de commande de fermeture Uferm au-dessus ou en-dessous de laquelle le débit de gaz Qve sortant par la valve expiratoire 7 est nul.

Par ailleurs, les moyens de mémorisation permettent également de mémoriser la valeur de tension de fermeture Uferm correspondant à l'instant donné t où le débit Q délivré par la source de gaz 2 est nul, c'est-à-dire lorsque Q = 0.

Les valeurs de tension U₁, U₂... Uₙ décroissantes ou croissantes délivrées par les moyens de pilotage 13 sont comprises entre la valeur de tension d'ouverture maximale Umax de la valve 7 jusqu'à la valeur de tension de fermeture Uferm, pour chaque valeur de pression de gaz successive P₁, P₂...Pₚ délivrée par la source de gaz 2.

De même, les valeurs de débit de gaz données (Q_{1,1}, Q_{1,2}... Q_{i,j}... Q_{n,p}) correspondant au débit sortant de la valve expiratoire 7 sont mesurées par le capteur de débit 9 pour chaque valeur de pression de gaz successive P₁, P₂...Pₚ du gaz délivré par la source de gaz 2 et pour chaque valeur de tension décroissantes U₁, U₂... Uₙ délivrée par les moyens de pilotage 13 .

Ensuite, les moyens de mémorisation, par exemple une mémoire type RAM, EEPROM ou Flash, permettent de mémoriser les valeurs de débit de gaz (Q_{1,1}, Q_{1,2}... Q_{i,j}... Q_{n,p}) de manière associée aux valeurs de pression de gaz successives P₁, P₂...Pₚ et auxdites valeurs de tensions croissantes ou décroissantes U₁, U₂... Uₙ correspondantes.

Les explications données ci-après permettent de mieux comprendre comment, à partir du modèle mis en place précédemment pendant l'autotest, on peut obtenir une estimation du débit de l'électrovanne Qve, y compris une information de débit nul, soit Qve =0. Ceci est le cas si la tension est inférieure ou supérieure (selon le mode de pilotage) à Uferm ou si les pressions en jeux au niveau de la valve sont telles qu'on sait qu'elle est fermée, c'est-à-dire si la pression proximale est inférieure à la pression de la baudruche/ballonnet de la valve.

### Autres mesures calculées

La seule pression mesurée par le capteur de pression 9 est la pression de sortie de la machine 1. Pour connaitre la pression à d'autre endroit du circuit 3, des modèles de calcul complémentaires peuvent être introduits pour déterminer par exemple :
- la pression proximale, c'est-à-dire avec prise en compte de la perte de charge du circuit patient, et
- la pression de la baudruche ou ballonnet de la valve expiratoire 7 qui est estimée en fonction de la pression délivrée par la turbine 2 et de la commande de la valve expiratoire 7.

Les modèles mis en place sont déterminés de façon empirique.

Si la pression de la baudruche est plus élevée que la pression proximale, alors le débit de la valve expiratoire est nul.

### Différents cas concernant l'estimation des fuites non intentionnelles

Une estimation des fuites non intentionnelles à la fin de l'expiration peut être mise en place avec les deux cas de figure suivants.

### a) Débit de la Valve expiratoire (Qve) nul

On sait que le débit Qve de la valve expiratoire est nul si la commande de la valve expiratoire descend en dessous d'un certain seuil déterminé lors des autotests ou si la pression proximale est plus faible que la pression baudruche.

Dans ce cas : Qve= 0

### b) Débit de la Valve expiratoire estimé par le tableau de caractérisation de l'autotest

Si on n'est pas dans le cas précédent, un débit Qve sort de la valve expiratoire 7. Ce débit Qve de la valve expiratoire 7 est estimé en faisant une double interpolation selon de la pression mesurée et de la commande de la valve expiratoire.

On a alors : Qve = Fonction (Pression, Tension de commande de la valve)

Les moyens de pilotage 13 calculent par ailleurs le débit Qve de gaz sortant de la valve expiratoire 7 à partir des valeurs de débit de gaz gaz (Q_{1,1}, Q_{1,2}... Q_{i,j}... Q_{n,p}) mesurées par le capteur de débit 9 et mémorisées par les moyens de mémorisation pour une valeur de tension instantanée Ui et pour une valeur de pression instantanée mesurée de gaz Pi, à l'aide d'une double interpolation sur une table (Ui, Pj, Qi,j) mémorisée au sein desdits moyens de mémorisation.

En outre, les moyens de pilotage 13 permettent de déterminer si le débit Qve de gaz sortant de la valve expiratoire 7 est nul à partir de la valeur de tension instantanée Ui à l'aide de la valeur de tension de fermeture Uferm mémorisée et des pression proximale et baudruche, c'est-à-dire du ballonnet la valve expiratoire 7.

### Utilisation

Le débit de valve expiratoire estimé peut alors servir à l'estimation des débits en jeux, lors de la ventilation.

Ainsi, une estimation du débit de fuite potentielle Qf entre l'appareil 1 et le patient 5 peut être effectuée par l'intermédiaire par exemple d'un modèle intégrant les valeurs de débit machine Q, de pression P et de débit de valve Qve, tel que :
Qf = F(Q, Qve, P)

De ce débit de fuite, peut être déduit le débit respiratoire du patient Qp, à savoir :
Qp = Q - Qf

Connaître le débit respiratoire du patient Qp est utile au médecin pour surveiller l'état de la ventilation et du patient. Le débit patient est utile au ventilateur pour calculer plusieurs monitorages comme par exemple, les volumes inspirés et expirés par le patient. Il sert aussi pour les régulations des modes volumétrique et des modes barométriques en asservissement en volume. Enfin, le débit patient est utilisé pour la synchronisation patient/machine par le biais du déclenchement (trigger) inspiratoire et du cyclage.

Par ailleurs, la Figure 2 représente un second mode de réalisation d'un appareil 1 de ventilation artificielle selon l'invention comprenant un circuit patient 3 comprenant une branche inspiratoire 3a et une branche expiratoire 3b, c'est-à-dire un circuit à double branche.

Dans ce cas, la branche inspiratoire 3a est reliée fluidiquement à la source de gaz 2 de manière à convoyer le gaz issu de la source de gaz 2 jusqu'à l'interface patient 4, comme dans le cas précédent, alors que la branche expiratoire 3b relie fluidiquement l'interface patient 4 à la valve expiratoire 7 agencée ici dans le ventilateur 1 de manière à convoyer le gaz expiré par le patient 5, c'est-à-dire les gaz riches en CO₂ expirés par le patient 5, et à l'expulser via la valve expiratoire 7.

Les branche inspiratoire 3a et expiratoire 3b sont reliées du côté de l'interface 4 par une pièce 13 en T ou en Y.

Dans ce deuxième mode de réalisation, la valve expiratoire 7 est agencée sur le circuit patient 3, à proximité du ou dans le ventilateur 1, en particulier en sortie de la branche expiratoire 3b au niveau du ventilateur 1.

Le fonctionnement de l'appareil dans ce second mode de réalisation est identique à celui du premier mode de réalisation.

## Revendications

1. Appareil de ventilation artificielle (1) d'un patient (5) comprenant :
- une source de gaz (2) apte à et conçue pour délivrer un gaz respiratoire,
- un circuit patient (3, 3a, 3b) comprenant au moins une branche inspiratoire (3a) reliée fluidiquement à la source de gaz (2) pour recevoir le débit de gaz respiratoire délivré par la source de gaz (2),
- une valve expiratoire (7), agencée sur ou reliée au circuit patient (3, 3a, 3b), dont l'ouverture et la fermeture sont commandées par une ligne de pressurisation (8) reliée fluidiquement à la source de gaz (2),
- au moins une électrovanne (6) agencée sur ladite ligne de pressurisation (8) et commandée par des moyens de pilotage (13) de manière à autoriser ou à interdire une transmission de pression à la valve expiratoire (7), via ladite ligne de pressurisation (8),
- des moyens de pilotage 13 conçus pour délivrer au moins un signal de tension (U) à ladite au moins une électrovanne (6) pour commander ladite au moins une électrovanne (6) de manière à autoriser ou interdire la transmission de pression à la valve expiratoire (7) au travers de ladite ligne de pressurisation (8) et à ainsi commander la valve expiratoire (7), en fonction dudit au moins un signal de tension (U), entre au moins une position totalement ouverte, une position totalement fermée et une ou plusieurs positions intermédiaires, et
- un moyen de mesure de pression (10) agencé de manière à mesurer la pression (P) du gaz délivré par la source de gaz (2),
**caractérisé en ce qu'**il comprend en outre des moyens à microprocesseur mettant en oeuvre au moins un algorithme permettant de déterminer le débit (Qve) de gaz sortant par la valve expiratoire (7) à partir d'au moins un signal de pression (P) délivré par le moyen de mesure de pression (10) et dudit au moins un signal de tension (U) délivré par les moyens de pilotage (13).

2. Appareil selon la revendication précédente, **caractérisé en ce qu'**il comprend en outre :
- un moyen de mesure de débit (9) agencé de manière à mesurer le débit (Q) du gaz délivré par la source de gaz (2), et
- les moyens de pilotage, le moyen de mesure de débit (9) et le moyen de mesure de pression (10) coopérant avec les moyens à microprocesseurs de manière à paramétrer l'algorithme des moyens à microprocesseurs servant à déterminer le débit (Qve) de gaz sortant par la valve expiratoire (7) pour une pression (P) et une tension (U) données.

3. Appareil selon l'une des revendications précédentes, **caractérisé en ce qu'**il comprend en outre des moyens de mémorisation (15) pour mémoriser au moins les valeurs de tension (U), de débit (Q) et de pression (P) issues des moyens de pilotage (13), du moyen de mesure de débit (9) et du moyen de mesure de pression (10), respectivement.

4. Appareil selon l'une des revendications précédentes, **caractérisé en ce que** les moyens à microprocesseurs coopèrent avec les moyens de mémorisation (15) de manière à retrouver des valeurs de tension (U), de débit (Q) et de pression (P) utilisées par l'algorithme pour estimer un débit de gaz (Qve) donné sortant par la valve expiratoire (7).

5. Appareil selon l'une des revendications précédentes, **caractérisé en ce que** les moyens à microprocesseurs sont conçus pour déterminer un débit de fuite (Qf) à partir de valeurs de débit de gaz (Qve) sortant par la valve expiratoire (7), et de débit (Q) et de pression (P) du gaz délivré par la source de gaz (2).

6. Appareil selon l'une des revendications précédentes, **caractérisé en ce que** les moyens de pilotage (13) sont aptes à et conçus pour délivrer des valeurs de tension (U) décroissantes ou croissantes à partir de la valeur de tension d'ouverture maximale (Umax) de la valve de manière à fermer progressivement la valve expiratoire (7).

7. Appareil selon l'une des revendications précédentes, **caractérisé en ce que**, lorsque la branche inspiratoire (3a) est obturée au niveau de l'interface (4) reliée au patient (5):
- la source de gaz (2) est apte à délivrer du gaz à pression donnée (P),
- les moyens de pilotage (13) sont programmés pour délivrer des valeurs de tension (U₁, U₂... Uₙ) décroissantes ou croissantes à partir de la valeur de tension d'ouverture maximale de la valve expiratoire (7) de manière à fermer progressivement ladite valve expiratoire (7),
- le capteur de débit (9) est apte à et conçu pour mesurer des valeurs de débit de gaz données (Q'₁, Q'₂... Q'ₙ) correspondant au débit de gaz sortant par la valve expiratoire (7), et
- les moyens à microprocesseurs permettent de déterminer la tension de commande de fermeture (Uferm) au-dessus ou en-dessous de laquelle le débit de gaz (Qve) sortant par la valve expiratoire (7) est nul.
- les moyens de mémorisation (15) sont aptes à et conçu pour mémoriser la valeur (Uferm).

8. Appareil selon l'une des revendications précédentes, **caractérisé en ce que**, lorsque la branche inspiratoire (3a) est obturée au niveau de l'interface (4) :
- la source de gaz (2) est apte à délivrer plusieurs valeurs de pression de gaz successives (P₁, P₂...Pj...Pₚ),
- les moyens de pilotage (13) sont programmés pour délivrer des valeurs de tension (U₁, U₂...Ui...Uₙ) décroissantes ou croissantes comprises depuis la valeur de tension d'ouverture maximale (Umax) de la valve (7) jusqu'à la valeur de tension de fermeture (Uferm), pour chaque valeur de pression de gaz successive (Pj) délivrée par la source de gaz (2),
- le capteur de débit (9) est apte à et conçu pour mesurer des valeurs de débit de gaz données (Q_{1,1}, Q_{1,2}... Q_{i,1}... Q_{n,p}) correspondant au débit sortant de la valve expiratoire pour chaque valeur de pression de gaz successive (P₁, P₂...Pₚ) du gaz délivré par la source de gaz (2) et pour chaque valeur de tensions décroissantes (U₁, U₂... Uₙ) délivrée par les moyens de pilotage (13), et
- les moyens de mémorisation (15) sont aptes à et conçu pour mémoriser les valeurs de débit de gaz (Q_{i,j}) mesurées par le capteur de débit (9) de manière associée aux valeurs de pression de gaz successives (P₁, P₂...Pj...Pₚ) et auxdites valeurs de tensions croissantes ou décroissantes (U₁, U₂...Ui...Uₙ) correspondantes.

9. Appareil selon l'une des revendications précédentes, **caractérisé en ce que** les moyens de pilotage (13) sont aptes à calculer le débit (Qve) de gaz sortant de la valve expiratoire (7) à partir des valeurs de débit de gaz (Q_{i,j}) mesurées par le capteur de débit (9) et mémorisées par les moyens de mémorisation (15) pour une valeur de tension instantanée (Ui) et pour une valeur de pression instantanée mesurée de gaz (Pj), à l'aide d'une double interpolation sur une table (Ui, Pj, Q_{i,j}) mémorisée au sein desdits moyens de mémorisation (15).

10. Appareil selon l'une des revendications précédentes, **caractérisé en ce que** les moyens de pilotage (13) sont aptes à déterminer si le débit (Qve) de gaz sortant de la valve expiratoire (7) est nul à partir de la valeur de tension instantanée (Ui) à l'aide de la valeur de tension de fermeture (Uferm) mémorisée.

11. Appareil selon l'une des revendications précédentes, **caractérisé en ce que** :
- le moyen de mesure de débit (9) est un capteur de débit, et/ou
- le moyen de mesure de pression (10) est un capteur de pression, et/ou
- les moyens de pilotage (13) comprennent une carte électronique, de préférence du type à microcontrôleur et algorithme, et/ou
- les moyens de mémorisation (15) comprennent au moins une mémoire type RAM, EEPROM ou Flash.

12. Appareil selon l'une des revendications précédentes, **caractérisé en ce que** les moyens de pilotage (13) sont conçus pour délivrer au moins un signal de tension (U) à l'électrovanne (6) pour commander l'électrovanne (6) de manière à autoriser ou interdire la transmission de pression à la valve expiratoire (7) au travers de ladite ligne de pressurisation (8) et à ainsi commander la valve expiratoire (7) entre au moins :
. une position totalement ouverte correspondant à une valeur de tension de commande maximale non nulle (Umax) de l'électrovanne (6) et dans laquelle le débit de gaz (Qve) sortant par la valve expiratoire (7) est maximal,
. une position totalement fermée correspondant à une valeur de tension de commande de fermeture (Uferm) de l'électrovanne (6), avec : Umax > Uferm ≥ 0, et dans laquelle le débit de gaz (Qve) sortant par la valve expiratoire (7) est nul,
. une ou plusieurs positions intermédiaire correspondant à une ou plusieurs valeurs de tension de commande intermédiaires (Uint) comprises entre Umax et Uferm, et dans laquelle ou lesquelles le débit de gaz (Qve) sortant par la valve expiratoire (7) est inférieur au débit maximal et non nul.

13. Appareil selon l'une des revendications précédentes, **caractérisé en ce que** les moyens de mémorisation (15) permettent de mémoriser la valeur de tension de fermeture (Uferm) correspondant à l'instant donné (t) où le débit (Q) délivré par la source de gaz (2) est nul (Q=0).

14. Appareil selon l'une des revendications précédentes, **caractérisé en ce que** la branche inspiratoire (3a) est reliée fluidiquement à une interface patient (4), de préférence l'interface patient (4) comprend un masque respiratoire, une sonde de trachéotomie ou une sonde d'intubation.

15. Appareil selon l'une des revendications précédentes, **caractérisé en ce que** la source de gaz (2) est une turbine, un récipient de gaz sous pression ou une ligne d'alimentation en gaz sous pression, de préférence une turbine (2).

16. Appareil selon l'une des revendications précédentes, **caractérisé en ce que** le circuit patient (3) comprenant une branche inspiratoire (3a) et une branche expiratoire (3b), la valve expiratoire (7) étant agencée sur la branche expiratoire (3b), de préférence la valve expiratoire (7) est agencée du côté de l'extrémité aval de la branche expiratoire (3b) située du côté de ou dans l'appareil (1).

## Patentansprüche

1. Gerät zur künstlichen Beatmung (1) eines Patienten (5), das Folgendes umfasst:
- eine Gasquelle (2), die geeignet und konzipiert ist, um ein Atemgas zu liefern,
- einen Patientenkreislauf (3, 3a, 3b), der mindestens einen Einatmungszweig (3a), der fluidtechnisch mit der Gasquelle (2) verbunden ist, umfasst, um den AtemgasDurchfluss, der von der Gasquelle (2) geliefert wird, zu empfangen,
- ein Ausatmungsventil (7), das auf dem Patientenkreislauf (3, 3a, 3b) angeordnet oder mit ihm verbunden ist, dessen Öffnen und Schließen von einer Druckbeaufschlagungsleitung (8), die fluidtechnisch mit der Gasquelle (2) verbunden ist, gesteuert werden,
- mindestens ein Magnetventil (6), das auf der Druckbeaufschlagungsleitung (8) angeordnet ist und von Steuermitteln (13) derart gesteuert wird, dass es eine Druckübertragung zu dem Ausatmungsventil (7) über die Druckbeaufschlagungsleitung (8) erlaubt oder verbietet.
- Steuermittel 13, die konzipiert sind, um mindestens ein Spannungssignal (U) zu dem mindestens einen Magnetventil (6) zu liefern, um das mindestens eine Magnetventil (6) derart zu steuern, dass die Druckübertragung zu dem Ausatmungsventil (7) über die Druckbeaufschlagungsleitung (8) erlaubt oder verboten wird und dadurch das Ausatmungsventil (7) in Abhängigkeit von dem mindestens einen Spannungssignal (U) zwischen mindestens einer vollständig offenen, einer vollständig geschlossenen und einer oder mehreren Zwischenpositionen zu steuern und,
- ein Druckmessmittel (10), das derart angeordnet ist, dass es den Druck (P) des von der Gasquelle (2) gelieferten Gases misst,
**dadurch gekennzeichnet, dass** es außerdem Mittel mit Mikroprozessor umfasst, die mindestens einen Algorithmus umsetzen, der es erlaubt, den Gasdurchfluss (Qve), der durch das Ausatmungsventil (7) austritt, ausgehend von mindestens einem Drucksignal (P), das von dem Druckmessmittel (10) geliefert wird, und dem mindestens einen Spannungssignal (U), das von den Steuermitteln (13) geliefert wird, zu bestimmen.

2. Gerät nach dem vorhergehenden Anspruch, **dadurch gekennzeichnet, dass** es ferner Folgendes umfasst:
- ein Durchflussmessmittel (9), das derart angeordnet ist, dass es den Durchfluss (Q) des Gases, das von der Gasquelle (2) geliefert wird, misst, und
- wobei die Steuermittel, dass Durchflussmessmittel (9) und das Druckmessmittel (10) mit den Mitteln mit Mikroprozessoren derart zusammenwirken, dass der Algorithmus der Mittel mit Mikroprozessoren, der dazu bestimmt ist, den Gasdurchfluss (Qve), der durch das Ausatmungsventil (7) austritt, für einen gegebenen Druck (P) und eine gegebene Spannung (U) zu bestimmen, parametriert wird.

3. Gerät nach einem der vorhergehenden Ansprüche, **dadurch gekennzeichnet, dass** es ferner Speichermittel (15) umfasst, um mindestens die Werte der Spannung (U), des Durchflusses (Q) und des Drucks (P), die jeweils aus den Steuermitteln (13), dem Durchflussmessmittel (9) und dem Druckmessmittel (10) stammen, zu speichern.

4. Gerät nach einem der vorhergehenden Ansprüche, **dadurch gekennzeichnet, dass** die Mittel mit Mikroprozessoren mit den Speichermitteln (15) derart zusammenwirken, dass Werte der Spannung (U), des Durchflusses (Q) und des Drucks (P), die von dem Algorithmus verwendet werden, um einen gegebenen Gasdurchfluss (Qve) zu schätzen, der durch das Ausatmungsventil (7) austritt, wieder gefunden werden.

5. Gerät nach einem der vorhergehenden Ansprüche, **dadurch gekennzeichnet, dass** die Mittel mit Mikroprozessoren konzipiert sind, um einen LeckDurchfluss (Qf) ausgehend von Werten (Qve) des Gasdurchflusses, der durch das Ausatmungsventil (7) austritt, und Durchflusses (Q) und Drucks (P) des Gases, das von der Gasquelle (2) geliefert wird, zu bestimmen.

6. Gerät nach einem der vorhergehenden Ansprüche, **dadurch gekennzeichnet, dass** die Steuermittel (13) geeignet und konzipiert sind, um Spannungswerte (U) zu liefern, die ausgehend von dem maximalen Öffnungsspannungswert (Umax) des Ventils derart sinken oder steigen, dass das Ausatmungsventil (7) allmählich geschlossen wird.

7. Gerät nach einem der vorhergehenden Ansprüche, **dadurch gekennzeichnet, dass**, wenn der Ausatmungszweig (3a) im Bereich der Schnittstelle (4), die mit dem Patienten (5) verbunden ist, verschlossen ist:
- die Gasquelle (2) fähig ist, Gase mit dem gegebenen Druck (P) zu liefern,
- die Steuermittel (13) programmiert sind, um Spannungswerte (U₁, U₂... Uₙ) zu liefern, die ausgehend von dem maximalen Öffnungsspannungswert des Ausatmungsventil (7) derart sinken oder steigen, dass das Ausatmungsventil (7) allmählich geschlossen wird,
- der Durchflusssensor (9) geeignet und konzipiert ist, um gegebene Gasdurchflusswerte (Q'₁; Q'₂... Q'ₙ), die dem Gasdurchfluss, der durch das Ausatmungsventil (7) austritt, entsprechen, zu messen, und
- die Mittel mit Mikroprozessoren es erlauben, die Schließsteuerspannung (Uferm) oberhalb oder unterhalb welcher der Gasdurchfluss (Qve), der durch das Ausatmungsventil (7) austritt, null ist, zu bestimmen,
- die Speichermittel (15) geeignet und konzipiert sind, um den Wert (Uferm) zu speichern.

8. Gerät nach einem der vorhergehenden Ansprüche, **dadurch gekennzeichnet, dass**, wenn der Einatmungszweig (3a) im Bereich der Schnittstelle (4) verschlossen ist:
- die Gasquelle (2) geeignet ist, mehrere aufeinanderfolgende Gasdruckwerte (P₁, P₂, ... Pⱼ... Pₚ) zu liefern,
- die Steuermittel (13) programmiert sind, um sinkende oder steigende Spannungswerte (U₁, U₂, ... Ui... Uₙ) zu liefern, die von dem maximalen Öffnungsspannungswert (Umax) des Ventils (7) bis zu dem Schließspannungswert (Uferm) für jeden aufeinanderfolgenden Gasdruckwert (Pⱼ), der von der Gasquelle (2) geliefert wird, reichen,
- der Durchflusssensor (9) geeignet und konzipiert ist, um gegebene Gasdurchflusswerte (Q_{1,1} Q_{1,2}... Q_{i,j}... Q_{n,p}), die dem Durchfluss entsprechen, der durch das Ausatmungsventil austritt, für jeden aufeinanderfolgenden Gasdruckwert (P₁, P₂... Pₚ) des Gases, das von der Gasquelle (2) geliefert wird, und für jeden Wert sinkender Spannungen (U₁, U₂... Uₙ), der von den Steuermitteln (13) geliefert wird, zu messen, und
- die Speichermittel (15) geeignet und konzipiert sind, um die Gasdurchflusswerte (Q_{i,j}), die von dem Durchflusssensor (9) verbunden mit den aufeinanderfolgenden Gasdruckwerten (P₁, P₂... P... Pₚ) und den entsprechenden steigenden oder Spannungswerten (U₁, U₂... Ui... Uₙ) gemessen werden, zu speichern.

9. Gerät nach einem der vorhergehenden Ansprüche, **dadurch gekennzeichnet, dass** die Steuermittel (13) geeignet sind, um den Gasdurchfluss (Qve), der durch das Ausatmungsventil (7) austritt, ausgehend von Gasdurchflusswerten (Q_{i,j}), die von dem Durchflusssensor (9) gemessen und von den Speichermitteln (15) gespeichert werden und für einen Momentanspannungswert (Ui) und für einen gemessenen Momentangasdruckwert (Pj) mit Hilfe einer doppelten Interpolation auf einer Tabelle (Uᵢ, Pⱼ, Q_{i,j}), die innerhalb der Speichermittel (15) gespeichert ist, zu berechnen.

10. Gerät nach einem der vorhergehenden Ansprüche, **dadurch gekennzeichnet, dass** die Steuermittel (13) geeignet sind zu bestimmen, ob der Gasdurchfluss (Qve), der durch das Ausatmungsventil (7) austritt, ausgehend von dem Momentanspannungswert (Ui) mit Hilfe des gespeicherten Schließspannungswerts (Uferm) null ist.

11. Gerät nach einem der vorhergehenden Ansprüche, **dadurch gekennzeichnet, dass**:
- das Durchflussmessmittel (9) ein Durchflussmesser ist, und/oder
- das Druckmessmittel (10) ein Drucksensor ist, und/oder
- die Steuermittel (13) eine Elektronikkarte, vorzugsweise des Typs mit Mikrocontroller und Algorithmus umfassen, und/oder
- die Speichermittel (15) mindestens einen Speicher des Typs RAM, EEPROM oder Flash umfassen.

12. Gerät nach einem der vorhergehenden Ansprüche, **dadurch gekennzeichnet, dass** die Steuermittel (13) konzipiert sind, um mindestens ein Spannungssignal (U) zu dem Magnetventil (6) zu liefern, um das Magnetventil (6) derart zu steuern, dass die Druckübertragung zu dem Ausatmungsventil (7) über die Druckbeaufschlagungsleitung (8) erlaubt oder verboten wird, und derart das Ausatmungsventil (7) mindestens zwischen Folgendem zu steuern:
- eine vollständig offene Position, die einem maximalen Steuerspannungswert nicht gleich null (Umax) des Magnetventils (6) entspricht und in der der Gasdurchfluss (Qve), der durch das Ausatmungsventil (7) austritt, maximal ist,
- eine vollständig geschlossene Position, die einem Schließsteuerspannungswert (Uferm) des Magnetventils (6) entspricht, wobei:
Umax > Uferm ≥ 0, und wobei der Gasdurchfluss (Qve), der durch das Ausatmungsventil (7) austritt, null ist,
- eine oder mehrere Zwischenpositionen, die einem oder mehreren Steuerspannungszwischenwerten (Uint) entsprechen, die zwischen Umax und Uferm liegen, und in der/den der Gasdurchfluss oder die Gasdurchsätze (Qve), der/die durch das Ausatmungsventil (7) austritt/austreten, kleiner ist/sind als der maximale Durchfluss und nicht gleich null.

13. Gerät nach einem der vorhergehenden Ansprüche, **dadurch gekennzeichnet, dass** die Speichermittel (15) es erlauben, den Schließspannungswert (Uferm), der dem gegebenen Augenblick (t), in dem der von der Gasquelle (2) gelieferte Durchfluss (Q) gleich null (Q=0) ist, zu speichern.

14. Gerät nach einem der vorhergehenden Ansprüche, **dadurch gekennzeichnet, dass** der Einatmungszweig (3a) fluidtechnisch mit einer Patientenschnittstelle (4) verbunden ist, die Patientenschnittstelle (4) vorzugsweise eine Atemmaske, eine Tracheotomiesonde oder einen Endotrachealtubus umfasst.

15. Gerät nach einem der vorhergehenden Ansprüche, **dadurch gekennzeichnet, dass** die Gasquelle (2) eine Turbine, ein Gasbehälter unter Druck oder eine Leitung zur Zuführung von Gas unter Druck, vorzugsweise eine Turbine (2) ist.

16. Gerät nach einem der vorhergehenden Ansprüche, **dadurch gekennzeichnet, dass** der Patientenkreislauf (3) einen Einatmungszweig (3a) und einen Ausatmungszweig (3b) umfasst, wobei das Ausatmungsventil (7) auf dem Ausatmungszweig (3b) angeordnet ist, wobei das Ausatmungsventil (7) bevorzugt auf der Seite des stromabwärtigen Endes des Ausatmungsventils (3b), der sich gerätseitig oder in dem Gerät (1) befindet, angeordnet ist.

## Claims

1. Artificial ventilation apparatus (1) for a patient (5) comprising;
- a gas source (2) suitable and designed for delivering a respiratory gas,
- a patient circuit (3, 3a, 3b) comprising at least one inspiratory branch (3a) fluidically connected to the gas source (2) to receive the respiratory gas flow delivered by the gas source (2),
- an expiratory valve (7), arranged on or connected to the patient circuit (3, 3a, 3b), the opening and closure whereof are controlled by a pressurisation line (8) fluidically connected to the gas source (2),
- at least one solenoid valve (6) arranged on said pressurisation line (8) and controlled by control means (13) so as to enable or disable transmission of pressure to the expiratory valve (7), via said pressurisation line (8),
- control means (13) designed for delivering at least one voltage signal (U) to said at least one solenoid valve (6) for controlling said at least one solenoid valve (6) so as to enable or disable transmission of pressure to the expiratory valve (7), via said pressurisation line (8) and thus control the expiratory valve (7), on the basis of said at least one voltage signal (U), between at least one fully open position, one fully closed position and one or a plurality of intermediate positions, and
- pressure measurement means (10) arranged so as to measure the pressure (P) of the gas delivered by the gas source (2),
**characterised in that** it further comprises microprocessor means using at least one algorithm for determining the flow rate (Qve) of gas outflowing via the expiratory valve (7) on the basis of at least one pressure signal (P) delivered by the pressure measurement means (10) and said at least one voltage signal (U) delivered by the control means (13).

2. Apparatus according to the above claim, **characterised in that** it further comprises:
- flow rate measurement means (9) arranged so as to measure the flow rate (Q) of the gas delivered by the gas source (2), and
- the control means, flow rate measurement means (9) and pressure measurement means (10) engaging with the microprocessor means so as to configure the algorithm of the microprocessor means used for determining the flow rate (Qve) of gas outflowing via the expiratory valve (7) for a given pressure (P) and voltage (U).

3. Apparatus according to any of the above claims, **characterised in that** it further comprises memory storage means (15) for storing in memory at least the voltage (U), flow rate (Q) and pressure (P) values obtained from the control means (13) flow rate measurement means (9) and pressure measurement means (10), respectively.

4. Apparatus according to any of the above claims, **characterised in that** the microprocessor means engage with the memory storage means (15) so as to retrieve the voltage (U), flow rate (Q) and pressure (P) values used by the algorithm to estimate a given gas flow rate (Qve) outflowing via the expiratory valve (7).

5. Apparatus according to any of the above claims, **characterised in that** the microprocessor means are designed for determining a leakage flow rate (Qf) on the basis of the values of the gas flow rate (Qve) outflowing via the expiratory valve (7), and flow rate (Q) and pressure (P) of the gas delivered by the gas source (2).

6. Apparatus according to any of the above claims, **characterised in that** the control means (13) are suitable and designed for delivering decreasing or increasing voltage values (U) on the basis of the maximum opening voltage value (Umax) of the valve so as to progressively close the expiratory valve (7).

7. Apparatus according to any of the above claims, **characterised in that**, when the inspiratory branch (3a) is occluded at the interface (4) connected to the patient (5):
- the gas source (2) is suitable for delivering the gas at a given pressure (P),
- the control means (13) are programmed to deliver decreasing or increasing voltage values (U₁, U₂... Uₙ) on the basis of the maximum opening voltage value (Umax) of the expiratory valve (7) so as to progressively close said expiratory valve (7),
- the flow rate sensor (9) is suitable and designed for measuring given gas flow rate values (Q'₁, Q'₂... Q'ₙ) corresponding to the gas flow rate outflowing via the expiratory valve (7), and
- the microprocessor means are suitable for determining the closing control voltage (Uferm) above and below which the gas flow rate (Qve) outflowing via the expiratory valve (7) is zero,
- the memory storage means (15) are suitable and designed for storing the value (Uferm) in memory.

8. Apparatus according to any of the above claims, **characterised in that**, when the inspiratory branch (3a) is occluded at the interface (4):
- the gas source (2) is suitable for delivering a plurality of successive gas pressure values (P₁, P₂... Pj...Pₚ),
- the control means (13) are programmed to deliver decreasing or increasing voltage values (U₁, U₂...Ui... Uₙ) included from the maximum opening voltage value (Umax) of the valve (7) to the closing voltage value (Uferm), for each successive gas pressure value (Pj) delivered by the gas source (2),
- the flow rate sensor (9) is suitable and designed for measuring given gas flow rate values (Q_{1,1}, Q_{1,2}... Q_{i,j}...Q_{n,p}) corresponding to the gas flow rate outflowing from the expiratory valve for each successive pressure value (P₁, P₂...Pₚ) of the gas delivered by the gas source (2) and for each decreasing voltage value (U₁, U₂... Uₙ) delivered by the control means (13), and
- the memory storage means (15) are suitable and designed for storing in memory the gas flow rate values (Q_{i,j}) measured by the flow rate sensor (9) in a manner associated with the successive gas pressure values (P₁, P₂ ... Pj ... Pₚ) and with said corresponding increasing or decreasing voltage values (U₁, U₂...Ui...Uₙ).

9. Apparatus according to any of the above claims, **characterised in that** the control means (13) are suitable for computing the flow rate (Qve) of gas outflowing from the expiratory valve (7) on the basis of the gas flow rate values (Q_{i,j}) measured by the flow rate sensor (9) and stored in memory by the memory storage means (15) for an instantaneous voltage value (Ui) and for an instantaneous measured gas value (Pj), using a double interpolation on a table (Ui, Pj, Qi,j) stored in memory within said memory storage means (15).

10. Apparatus according to any of the above claims, **characterised in that** the control means (13) are suitable for determining whether the flow rate (Qve) of gas outflowing from the expiratory valve (7) is zero on the basis of the instantaneous voltage value (Ui) using the closing voltage value (Uferm) stored in memory.

11. Apparatus according to any of the above claims, **characterised in that**:
- the flow rate measurement means (9) are a flow rate sensor, and/or
- the pressure measurement means (10) are a pressure sensor, and/or
- the control means (13) comprise an electronic board, preferably of the microcontroller and algorithm type, and/or
- the memory storage means (15) comprise at least at least one RAM, EEPROM or Flash type memory.

12. Apparatus according to any of the above claims, **characterised in that** the control means (13) are designed for delivering at least one voltage signal (U) to the solenoid valve (6) for controlling said at least one solenoid valve (6) so as to enable or disable transmission of pressure to the expiratory valve (7), via said pressurisation line (8) and thus control the expiratory valve (7) between at least:
. a fully open position corresponding to a maximum control voltage value different to zero (Umax) of the solenoid valve (6) and wherein the gas flow rate (Qve) outflowing via the expiratory valve (7) is maximum,
. a fully closed position corresponding to a closing control voltage value (Uferm) of the solenoid valve (6), where:
Umax > Uferm ≥ 0, and wherein the gas flow rate (Qve) outflowing via the expiratory valve (7) is zero,
. one or a plurality of intermediate positions corresponding to one or a plurality of intermediate control voltage values (Uint) between Umax and Uferm, and wherein the gas flow rate (Qve) outflowing via the expiratory valve (7) is less than the maximum flow rate and different to zero.

13. Apparatus according to any of the above claims, **characterised in that** the memory storage means (15) are suitable for storing in memory the closing voltage value (Uferm) corresponding to the given time (t) where the flow rate (Q) delivered by the gas source (2) is zero (Q=0).

14. Apparatus according to any of the above claims, **characterised in that** the inspiratory branch (3a) is fluidically connected to a patient interface (4), preferably the patient interface (4) comprises a breathing mask, a tracheotomy tube or an intubation tube.

15. Apparatus according to any of the above claims, **characterised in** the gas source (2) is a turbine, a pressurised gas container or a pressurised gas supply line, preferably a turbine (2).

16. Apparatus according to any of the above claims, **characterised in that** the patient circuit (3) comprising an inspiratory branch (3a) and an expiratory branch (3b), the expiratory valve (7) being arranged on the expiratory branch (3b), preferably the expiratory valve (7) is arranged on the side of the downstream end of the expiratory branch (3b) situated on the side of or in the apparatus (1).
